# EUROPEAN PATENT APPLICATION

(11) **EP 4 821 486 A1**
(43) Date of publication of application: **14.10.2026**
(21) Application number: 24902796.2
(22) Date of filing: 10.12.2024
(51) Int. Cl.: A61K 9/28, A61K 9/20, A61K 9/22, A61K 31/4015, A61K 47/38, A61P 25/08

(54) **BRIVARACETAM SUSTAINED-RELEASE PHARMACEUTICAL COMPOSITION, PREPARATION METHOD THEREFOR, AND USE THEREOF**

(30) Priority: 10.12.2023 CN 202311691841; 05.12.2024 CN 202411783409
(71) Applicant: Shanghai Yunsanova Biotechnology Co., Ltd., Shanghai 201203 (CN)
(72) Inventor: YING, Shuhuan, Shanghai 201203 (CN); GUO, Zhen, Shanghai 201203 (CN); XIE, Wenfeng, Shanghai 201203 (CN); XIAO, Xiaoling, Shanghai 201203 (CN); WANG, Tingting, Shanghai 201203 (CN)
(74) Representative: Gille Hrabal Partnerschaftsgesellschaft mbB Patentanwälte
(86) International application number: PCT/CN2024/138119
(87) International publication number: WO 2025/124375

(57) **Abstract**

A brivaracetam sustained-release pharmaceutical composition, a preparation method therefor, and use thereof. The brivaracetam sustained-release pharmaceutical composition comprises an active pharmaceutical ingredient and a pharmaceutical excipient. The pharmaceutical excipient is selected from one or more of a matrix material, a disintegrating agent, a swelling agent, a binder, a filler, and a lubricant.

## Description

The present application claims priority to the following two prior applications: the prior application with the patent application No. 202311691841.7 filed with China National Intellectual Property Administration on Dec. 10, 2023 and entitled "BRIVARACETAM SUSTAINED-RELEASE PHARMACEUTICAL COMPOSITION, PREPARATION METHOD THEREFOR, AND USE THEREOF" and the prior application with the patent application No. 202411783409.5 filed with China National Intellectual Property Administration on Dec. 5, 2024 and entitled "BRIVARACETAM SUSTAINED-RELEASE PHARMACEUTICAL COMPOSITION, PREPARATION METHOD THEREFOR, AND USE THEREOF", which are incorporated herein by reference in their entirety.

### TECHNICAL FIELD

The present disclosure pertains to the field of pharmaceutical compositions, and relates to a brivaracetam sustained-release pharmaceutical composition, a preparation method therefor, and use thereof.

### BACKGROUND

Epilepsy is a chronic brain disease, which is a temporary brain dysfunction caused by highly synchronous firing of neurons in the brain and is commonly known in Chinese folk nomenclature as "Yang Jiao Feng" or "Yang Dian Feng". Currently, epilepsy has become the second most common disease of the nervous system in China. According to the latest epidemiological data from China, there are about 9 million epilepsy patients in China, of which 5-6 million are active epilepsy patients, and about 400,000 new epilepsy patients are diagnosed annually (https://www.caae.org.cn/news/show/id/294). Epileptic seizures affect the daily work and life of patients, and are mainly manifested by mental disorders such as motor, sensory, autonomic, and consciousness disorders, cognitive dysfunctions such as memory impairment, attention reduction, and mental decline, and emotional disorders such as anxiety and depression. Long-term and frequent seizures have serious impacts on the physical and mental health of patients and may also cause accidental injuries to patients. Since the emergence of the first drug for treating epilepsy, phenobarbital, in 1912, antiepileptic drugs have developed rapidly. Currently, antiepileptic drugs may be divided into 3 generations: (1) traditional antiepileptic drugs, including phenytoin sodium, carbamazepine, sodium valproate, and the like; (2) modern antiepileptic drugs, including oxcarbazepine, levetiracetam, gabapentin, and the like; and (3) novel antiepileptic drugs, including brivaracetam, carabersat, retigabine, perampanel, and the like. The therapeutic drugs commonly used clinically include phenytoin sodium, phenobarbital, primidone, carbamazepine, sodium valproate, and the like.

Brivaracetam, an antiepileptic drug developed by UCB, is suitable for the treatment of partial-onset seizures. Brivaracetam has the chemical name of (2S)-2-[(4R)-2-oxo-4-propyltetrahydro-1H-pyrrol-1-yl] butanamide and the molecular formula of C₁₁H₂₀N₂O₂. Its structural formula is as follows:

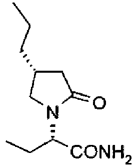

Brivaracetam exhibits selectivity and high affinity for synaptic vesicle glycoprotein 2A (SV2A) in the brain. SV2A is a transmembrane glycoprotein found at the presynaptic level in neurons and endocrine cells. Although the exact role of this protein has yet to be elucidated, it has been shown that this protein can regulate the exocytosis of neurotransmitters. The binding to SV2A should be the main mechanism for the anticonvulsant activity of brivaracetam. The marketing of brivaracetam constitutes a new addition to the adjuvant therapy for partial-onset seizures, particularly for patients whose epileptic seizures are inadequately controlled with one or more additional adjuvant therapies.

The dosage forms of brivaracetam currently on the market are common oral tablets, oral solutions, and injectable liquids. The oral dosage forms feature frequent administrations, great fluctuations in plasma concentration, and relatively serious adverse effects, and the injectable liquid dosage form features complex medication and relatively poor compliance.

Therefore, there is an urgent need to develop a sustained-release tablet to achieve the purposes of reducing the drug release rate, controlling the effective dose of the drug in the body, and reducing the daily frequency of administration.

### SUMMARY

The technical problem that the present disclosure solves is to provide a brivaracetam sustained-release pharmaceutical composition that is different from the prior art and exhibits a good sustained-release effect, stable plasma concentration, good efficacy, mild side effects, and good compliance, a preparation method therefor, and use thereof.

In order to solve the technical problem described above, the present disclosure provides a brivaracetam sustained-release pharmaceutical composition, which is a 24-hour sustained-release drug, and the dissolution of the pharmaceutical composition simultaneously meets the following four features (determined according to Method 7 in General Chapter 0931, Dissolution and Drug Release Test, of Chinese Pharmacopoeia, 2020 Edition; dissolution conditions: the dissolution medium is a hydrochloric acid solution at pH 1.2 and/or an acetate buffer at pH 4.5, the immersion speed is 20 DPM, the sieve is a 20-mesh polypropylene sieve, and the dripping time is 15 seconds):
A) no more than 40% of an active pharmaceutical ingredient is dissolved within 1 hour;
B) 20%-70% of the active pharmaceutical ingredient is dissolved within 4 hours;
C) 60%-90% of the active pharmaceutical ingredient is dissolved within 12 hours; and
D) no less than 70% of the active pharmaceutical ingredient is dissolved within 24 hours;
the active pharmaceutical ingredient may be selected from one, two, or more of brivaracetam, a pharmaceutically acceptable complex of brivaracetam, a pharmaceutically acceptable salt of brivaracetam, a pharmaceutically acceptable solvate of brivaracetam, and a pharmaceutically acceptable hydrate of brivaracetam.

According to some embodiments of the present disclosure, the brivaracetam sustained-release pharmaceutical composition is a 24-hour sustained-release drug, and the dissolution of the pharmaceutical composition simultaneously meets the following four features:
A) no more than 35% of the active pharmaceutical ingredient is dissolved within 1 hour;
B) 30%-70% of the active pharmaceutical ingredient is dissolved within 4 hours;
C) 60%-85% of the active pharmaceutical ingredient is dissolved within 12 hours; and
D) no less than 75% of the active pharmaceutical ingredient is dissolved within 24 hours.

According to some embodiments of the present disclosure, the brivaracetam sustained-release pharmaceutical composition is a 24-hour sustained-release drug, and the dissolution of the pharmaceutical composition simultaneously meets the following four features:
A) no more than 30% of the active pharmaceutical ingredient is dissolved within 1 hour; preferably, no more than 30% of brivaracetam or the pharmaceutically acceptable salt thereof is dissolved within 1 hour;
B) 35%-60% of the active pharmaceutical ingredient is dissolved within 4 hours; preferably, 35%-60% of brivaracetam or the pharmaceutically acceptable salt thereof is dissolved within 4 hours;
C) 65%-85% of the active pharmaceutical ingredient is dissolved within 12 hours; preferably, 65%-85% of brivaracetam or the pharmaceutically acceptable salt thereof is dissolved within 12 hours; and
D) no less than 80% of the active pharmaceutical ingredient is dissolved within 24 hours; preferably, no less than 80% of brivaracetam or the pharmaceutically acceptable salt thereof is dissolved within 24 hours.

The "dissolution" refers to the cumulative dissolution amount of the active pharmaceutical ingredient (e.g., brivaracetam or the pharmaceutically acceptable salt thereof); further, the cumulative dissolution amount is measured in a hydrochloric acid solution at pH 1.2 or an acetate buffer at pH 4.5. Those skilled in the art will appreciate that the dissolution of brivaracetam or the pharmaceutically acceptable salt thereof will gradually increase over time.

The present disclosure provides a brivaracetam sustained-release pharmaceutical composition comprising an active pharmaceutical ingredient and a pharmaceutical excipient, wherein the pharmaceutical excipient is selected from one or more of a skeleton material, a disintegrant, a sweller, an adhesive, a filler, and a lubricant; the active pharmaceutical ingredient is selected from one or more of the following: brivaracetam, a pharmaceutically acceptable complex of brivaracetam, a pharmaceutically acceptable salt of brivaracetam, a pharmaceutically acceptable solvate of brivaracetam, and a pharmaceutically acceptable hydrate of brivaracetam;
preferably, the brivaracetam sustained-release pharmaceutical composition has the dissolution features as shown above.

According to an embodiment of the present disclosure, the brivaracetam sustained-release pharmaceutical composition comprises microcrystalline cellulose and/or hydroxypropyl methylcellulose;
in some embodiments, the microcrystalline cellulose has a weight percentage of 1.00%-60.00%, for example, 2.00%-40.00%, for another example, 5.00%-15.00%, illustratively 2.00%, 3.00%, 4.00%, 5.00%, 5.55%, 6.00%, 7.00%, 8.00%, 8.09%, 9.00%, 10.00%, 10.09%, 11.00%, 12.00%, 13.00%, 14.00%, 15.00%, 20.00%, 25.00%, 30.00%, 35.00%, 40.00%, 50.00%, or 60.00%; the weight percentage refers to a percentage of the weight of the microcrystalline cellulose in the total weight of the brivaracetam sustained-release pharmaceutical composition;
in some embodiments, the hydroxypropyl methylcellulose has a weight percentage of 1.00%-40.00%, for example, 2.00%-20.00%, illustratively 2.00%, 3.00%, 3.45%, 4.00%, 5.00%, 5.09%, 6.00%, 7.00%, 8.00%, 8.09%, 9.00%, 9.09%, 10.00%, 11.00%, 12.00%, 13.00%, 14.00%, 15.00%, 20.00%, 25.00%, 30.00%, 35.00%, or 40.00%; the weight percentage refers to a percentage of the weight of the hydroxypropyl methylcellulose in the total weight of the brivaracetam sustained-release pharmaceutical composition.

According to an embodiment of the present disclosure, the active pharmaceutical ingredient is preferably brivaracetam.

According to an embodiment of the present disclosure, the active pharmaceutical ingredient has a weight percentage of preferably 2.00%-50.00%, and further preferably 3.00%-20.00%, for example, 4.00%, 4.55%, 5.00%, 6.00%, 7.00%, 8.00%, 9.00%, 9.09%, 10.00%, 11.00%, 12.00%, 13.00%, 13.64%, 14.00%, 15.00%, 16.00%, 17.00%, 18.00%, 18.18%, 19.00%, 20.00%, 25.00%, 30.00%, 35.00%, 40.00%, or 50.00%; the weight percentage refers to a percentage of the weight of the active pharmaceutical ingredient in the total weight of the brivaracetam sustained-release pharmaceutical composition.

According to an embodiment of the present disclosure, the skeleton material refers to a substance capable of providing structural integrity and helping control or prolong the drug release rate, and is selected from one or more of polyvinyl acetate povidone mixture (Kollidon^{®}SR, hereinafter referred to as KSR), sodium alginate, ethylcellulose, cellulose acetate, cellulose acetate butyrate, poloxamer, polacrilin potassium, acrylic resin, and polyvinyl alcohol, preferably KSR. The KSR may be available from BASF under the trade name KOLLIDON^{@}SR, and comprises an 80/20 (w/w) mixture of PVAc and PVP.

According to an embodiment of the present disclosure, the skeleton material has a weight percentage of preferably 5.00%-60.00%, and further preferably 20.00%-40.00%, for example, 20.00%, 25.00%, 29.10%, 30.00%, 30.27%, 31.37%, 32.00%, 33.00%, 34.00%, 35.00%, or 40.00%; the weight percentage refers to a percentage of the weight of the skeleton material in the total weight of the brivaracetam sustained-release pharmaceutical composition.

According to an embodiment of the present disclosure, the disintegrant refers to an auxiliary material for promoting rapid disintegration of a drug into small particles in the gastrointestinal tract, and is selected from one or more of sodium carboxymethyl starch, low-substituted hydroxypropylcellulose, corn starch, and croscarmellose sodium, preferably sodium carboxymethyl starch.

According to an embodiment of the present disclosure, the disintegrant has a weight percentage of preferably 1.00%-10.00%, and further preferably 2.00%-8.00%, for example, 3.00%, 4.00%, 5.00%, 5.18%, 6.00%, 6.10%, 6.36%, or 7.00%; the weight percentage refers to a percentage of the weight of the disintegrant in the total weight of the brivaracetam sustained-release pharmaceutical composition.

According to an embodiment of the present disclosure, the sweller refers to a substance capable of absorbing water from gastric juice to enable the expansion of the size of a solid formulation and affecting the drug release rate by generating channels or forming hydrophilic colloids; the sweller is soluble or insoluble in water and is selected from one or more of polyoxyethylene (PEO), crospovidone, carbomer, sodium carboxymethylcellulose, and calcium carboxymethylcellulose, preferably one or more of polyoxyethylene, crospovidone, and carbomer, and more preferably polyoxyethylene and/or crospovidone. The polyoxyethylene may be a conventional commercially available polyoxyethylene product, such as polyoxyethylene N60K and/or polyoxyethylene 1105.

According to an embodiment of the present disclosure, the sweller has a weight percentage of preferably 5.00%-60.00%, and further preferably 10.00%-40.00%, for example, 10.00%, 15.00%, 17.00%, 19.00%, 20.00%, 23.00%, 25.00%, 27.00%, 28.18%, 30.00%, 30.36%, 30.72%, 33.00%, 35.00%, or 40.00%; the weight percentage refers to a percentage of the weight of the sweller in the total weight of the brivaracetam sustained-release pharmaceutical composition.

According to an embodiment of the present disclosure, the adhesive refers to a conventional additive in the art that is capable of increasing the viscosity of a dispersion medium to reduce the settling rate of microparticles or increase the hydrophilicity of microparticles, and is selected from one or more of hydroxypropyl methylcellulose, pregelatinized starch, copovidone, hydroxyethylcellulose, and dextrin, preferably hydroxypropyl methylcellulose and/or pregelatinized starch.

According to an embodiment of the present disclosure, the adhesive has a weight percentage of preferably 1.00%-40.00%, and further preferably 5.00%-20.00%, for example, 2.00%, 3.00%, 4.00%, 5.00%, 7.00%, 8.00%, 10.00%, 11.63%, 15.00%, 15.09%, 16.00%, 19.09%, 20.00%, 21.00%, 22.00%, 23.00%, 24.00%, 25.00%, or 30.00%; the weight percentage refers to a percentage of the weight of the adhesive in the total weight of the brivaracetam sustained-release pharmaceutical composition.

According to an embodiment of the present disclosure, the filler refers to a solid substance capable of improving the performance of a material, increasing the volume and weight, and reducing the cost of the material when added to the material, and is selected from one or more of microcrystalline cellulose, silicified microcrystalline cellulose, lactose (e.g., a lactose hydrate or anhydrous lactose, wherein the lactose hydrate is, for example, lactose monohydrate), mannitol, and sorbitol, preferably microcrystalline cellulose and/or lactose. According to an embodiment of the present disclosure, the filler has a weight percentage of preferably 1.00%-60.00%, and further preferably 5.00%-40.00%, for example, 2.00%, 3.00%, 4.00%, 5.00%, 5.55%, 6.00%, 7.00%, 8.00%, 8.09%, 9.00%, 10.00%, 10.09%, 15.00%, 19.00%, 20.00%, 25.00%, 28.91%, 30.00%, 32.91%, 35.00%, or 40.00%; the weight percentage refers to a percentage of the weight of the filler in the total weight of the brivaracetam sustained-release pharmaceutical composition.

According to an embodiment of the present disclosure, the lubricant refers to a substance that facilitates processing steps such as mixing of components and tableting, and is selected from one or more of magnesium stearate, calcium stearate, and sodium stearyl fumarate, preferably magnesium stearate.

According to an embodiment of the present disclosure, the lubricant has a weight percentage of preferably 1.00%-5.00%, and further preferably 1.00%-2.00%, for example, 1.00%; the weight percentage refers to a percentage of the weight of the lubricant in the total weight of the brivaracetam sustained-release pharmaceutical composition.

According to some embodiments of the present disclosure, the brivaracetam sustained-release pharmaceutical composition preferably consists of an active pharmaceutical ingredient, a skeleton material, a disintegrant, a sweller, an adhesive, a filler, and a lubricant;
preferably, the active pharmaceutical ingredient is brivaracetam or a pharmaceutically acceptable salt thereof;
and/or, the skeleton material is polyvinyl acetate povidone mixture;
and/or, the disintegrant is sodium carboxymethyl starch;
and/or, the sweller is selected from one or more of polyoxyethylene, crospovidone, and carbomer;
and/or, the adhesive is selected from hydroxypropyl methylcellulose and/or pregelatinized starch;
and/or, the filler is selected from microcrystalline cellulose and/or lactose;
and/or, the lubricant is selected from magnesium stearate.

In some embodiments, the brivaracetam sustained-release pharmaceutical composition consists of the following components in percentage by weight: 2.00%-50.00% of brivaracetam, 5.00%-60.00% of the skeleton material, 1.00%-10.00% of the disintegrant, 5.00%-60.00% of the sweller, 1.00%-40.00% of the adhesive, 1.00%-60.00% of the filler, and 1.00%-5.00% of the lubricant, wherein the percentage by weight refers to a percentage of the weight of an individual component in the total weight of the brivaracetam sustained-release pharmaceutical composition.

In some embodiments, the brivaracetam sustained-release pharmaceutical composition consists of the following components in percentage by weight: 3.00%-20.00% of brivaracetam, 20.00%-40.00% of the skeleton material, 2.00%-8.00% of the disintegrant, 10.00%-40.00% of the sweller, 5.00%-20.00% of the adhesive, 5.00%-40.00% of the filler, and 1.00%-2.00% of the lubricant, wherein the percentage by weight refers to a percentage of the weight of an individual component in the total weight of the brivaracetam sustained-release pharmaceutical composition.

According to an embodiment of the present disclosure, the brivaracetam sustained-release pharmaceutical composition preferably consists of the following formulations:
formulation 1: brivaracetam, KSR, crospovidone, carbomer, lactose, hydroxypropyl methylcellulose, and magnesium stearate;
formulation 2: brivaracetam, KSR, crospovidone, carbomer, lactose, hydroxypropyl methylcellulose, microcrystalline cellulose, and magnesium stearate;
formulation 3: brivaracetam, KSR, crospovidone, polyoxyethylene, microcrystalline cellulose, hydroxypropyl methylcellulose, and magnesium stearate;
formulation 4: brivaracetam, KSR, sodium carboxymethyl starch, crospovidone, polyoxyethylene, microcrystalline cellulose, pregelatinized starch, hydroxypropyl methylcellulose, and magnesium stearate;
formulation 5: brivaracetam, KSR, sodium carboxymethyl starch, crospovidone, polyoxyethylene (e.g., polyoxyethylene N60K and/or polyoxyethylene 1105), microcrystalline cellulose, pregelatinized starch, hydroxypropyl methylcellulose, and magnesium stearate.

According to an embodiment of the present disclosure, the brivaracetam sustained-release pharmaceutical composition may be selected from any one of the following formulas:
formula 1: 9.09% brivaracetam, 30.00% polyvinyl acetate povidone mixture, 15.00% crospovidone, 4.00% carbomer, 8.00% hydroxypropyl methylcellulose, 32.91% lactose monohydrate, and 1.00% magnesium stearate;
formula 2: 9.09% brivaracetam, 30.00% polyvinyl acetate povidone mixture, 15.00% crospovidone, 8.00% carbomer, 8.00% hydroxypropyl methylcellulose, 20.91% lactose monohydrate, 8.00% microcrystalline cellulose, and 1.00% magnesium stearate;
formula 3: 10.00% brivaracetam, 30.00% polyvinyl acetate povidone mixture, 17.00% crospovidone, 16.00% polyoxyethylene 1105, 7.00% hydroxypropyl methylcellulose, 19.00% microcrystalline cellulose, and 1.00% magnesium stearate;
formula 4: 10.00% brivaracetam, 32.00% polyvinyl acetate povidone mixture, 5.00% sodium carboxymethyl starch, 12.00% crospovidone, 15.00% polyoxyethylene N60K, 7.00% hydroxypropyl methylcellulose, 8.00% pregelatinized starch, 10.00% microcrystalline cellulose, and 1.00% magnesium stearate;
formula 5: 10.00% brivaracetam, 32.00% polyvinyl acetate povidone mixture, 5.00% sodium carboxymethyl starch, 12.00% crospovidone, 7.50% polyoxyethylene N60K, 7.50% polyoxyethylene 1105, 8.00% hydroxypropyl methylcellulose, 8.00% pregelatinized starch, 9.00% microcrystalline cellulose, and 1.00% magnesium stearate;
formula 6: 10.00% brivaracetam, 30.00% polyvinyl acetate povidone mixture, 6.00% sodium carboxymethyl starch, 15.00% crospovidone, 15.00% polyoxyethylene N60K, 5.00% hydroxypropyl methylcellulose, 10.00% pregelatinized starch, 8.00% microcrystalline cellulose, and 1.00% magnesium stearate;
formula 7: 9.09% brivaracetam, 30.27% polyvinyl acetate povidone mixture, 6.10% sodium carboxymethyl starch, 15.18% crospovidone, 15.18% polyoxyethylene N60K, 5.09% hydroxypropyl methylcellulose, 10.00% pregelatinized starch, 8.09% microcrystalline cellulose, and 1.00% magnesium stearate;
formula 8: 4.55% brivaracetam, 31.37% polyvinyl acetate povidone mixture, 5.18% sodium carboxymethyl starch, 14.36% crospovidone, 16.36% polyoxyethylene N60K, 9.09% hydroxypropyl methylcellulose, 10.00% pregelatinized starch, 8.09% microcrystalline cellulose, and 1.00% magnesium stearate;
formula 9: 13.64% brivaracetam, 29.10% polyvinyl acetate povidone mixture, 6.36% sodium carboxymethyl starch, 15.45% crospovidone, 12.73% polyoxyethylene N60K, 3.45% hydroxypropyl methylcellulose, 8.18% pregelatinized starch, 10.09% microcrystalline cellulose, and 1.00% magnesium stearate;
formula 10: 18.18% brivaracetam, 29.10% polyvinyl acetate povidone mixture, 6.36% sodium carboxymethyl starch, 15.45% crospovidone, 12.73% polyoxyethylene N60K, 3.45% hydroxypropyl methylcellulose, 8.18% pregelatinized starch, 5.55% microcrystalline cellulose, and 1.00% magnesium stearate;
the percentage refers to a percentage of the weight of each component in the total weight of the brivaracetam sustained-release pharmaceutical composition.

The present disclosure further provides a brivaracetam sustained-release tablet, comprising the brivaracetam sustained-release pharmaceutical composition described above.

Preferably, the brivaracetam sustained-release tablet consists of a tablet core and a coating agent, wherein the tablet core comprises the brivaracetam sustained-release pharmaceutical composition described above.

According to an embodiment of the present disclosure, the coating agent is preferably a film coating powder.

The content of the coating agent is preferably 0%-10.0%, for example, 3.00% or 4.00%, wherein the content refers to a percentage of the weight of the coating agent in the total weight of the tablet core.

According to an embodiment of the present disclosure, the tablet core consists of the following components in percentage by weight: 2.00%-50.00% of brivaracetam, 5.00%-60.00% of the skeleton material, 1.00%-10.00% of the disintegrant, 5.00%-60.00% of the sweller, 1.00%-40.00% of the adhesive, 1.00%-60.00% of the filler, and 1.00%-5.00% of the lubricant, wherein the percentage by weight refers to a percentage of the weight of an individual component in the total weight of the brivaracetam sustained-release tablet.

According to an embodiment of the present disclosure, the tablet core consists of the following components in percentage by weight: 3.00%-20.00% of brivaracetam, 20.00%-40.00% of the skeleton material, 2.00%-8.00% of the disintegrant, 10.00%-40.00% of the sweller, 5.00%-20.00% of the adhesive, 5.00%-40.00% of the filler, and 1.00%-2.00% of the lubricant, wherein the percentage by weight refers to a percentage of the weight of an individual component in the total weight of the brivaracetam sustained-release tablet.
preferably, the brivaracetam sustained-release pharmaceutical composition has the dissolution features as shown above.

The present disclosure further provides a preparation method for the brivaracetam sustained-release pharmaceutical composition or the brivaracetam sustained-release tablet, wherein the preparation method may be a direct powder compression method, a dry granulation method, a wet granulation method, or a fluidized granulation method.

For example, the preparation method for the brivaracetam sustained-release pharmaceutical composition or the brivaracetam sustained-release tablet comprises: mixing and sieving the components other than the lubricant (e.g., the active pharmaceutical ingredient, the matrix-forming agent, and the sweller) in the sustained-release pharmaceutical composition, then mixing with the lubricant, and tableting to give a tablet, i.e., the brivaracetam sustained-release composition, wherein
the tablet may be further coated to give a coated tablet, i.e., the brivaracetam sustained-release tablet.

The brivaracetam sustained-release pharmaceutical composition or the brivaracetam sustained-release tablet described in the present disclosure comprises 25 mg to 200 mg, preferably 25 mg, 50 mg, 100 mg, 150 mg, or 200 mg, of brivaracetam.

The present disclosure further provides use of the brivaracetam sustained-release pharmaceutical composition for manufacturing a medicament. Further, the medicament is used for treating and/or preventing the following conditions or diseases: epilepsy (e.g., partial-onset seizures), Parkinson's disease, dyskinesia, migraine, tremor, essential tremor, bipolar disorder, chronic diseases, neuropathic pain, bronchial asthma, allergic bronchial asthma, or the like. In some embodiments, the medicament is the brivaracetam sustained-release tablet.

The brivaracetam sustained-release pharmaceutical composition or the brivaracetam sustained-release tablet described in the present disclosure is particularly suitable for treating partial-onset seizures in patients aged 16 years or more.

The present disclosure further provides use of the brivaracetam sustained-release pharmaceutical composition for manufacturing a pharmaceutical formulation. The pharmaceutical formulation may be a common oral formulation, and the dosage form of the common oral formulation includes an oral tablet. In some embodiments, the pharmaceutical formulation is the brivaracetam sustained-release tablet.

The present disclosure further provides a method for treating a condition in a patient responsive to brivaracetam, the method comprising orally administering to the patient the brivaracetam sustained-release pharmaceutical composition or the brivaracetam sustained-release tablet described above once daily.

The present disclosure further provides a method for preventing and/or treating a condition or disease, comprising administering to a patient a therapeutically effective amount of the brivaracetam sustained-release pharmaceutical composition or the brivaracetam sustained-release tablet described above, for example, orally administering to the patient the brivaracetam sustained-release pharmaceutical composition or the brivaracetam sustained-release tablet described above once daily, wherein
preferably, the condition or disease has the definition described above.

The brivaracetam sustained-release pharmaceutical composition or the brivaracetam sustained-release tablet described in the present disclosure has stable properties and is suitable for being orally administered once daily. The sustained-release pharmaceutical composition, when administered in a solid dosage form, has a longer retention time in the stomach than an immediate-release formulation. The sustained-release pharmaceutical composition, when retained in the stomach, can continuously release brivaracetam. When the brivaracetam sustained-release pharmaceutical composition or the brivaracetam sustained-release tablet is taken integrally and enters the stomach of a patient, it can float rapidly in the gastric juice and slowly expand or swell. The formulation, when expanding to a certain size, can prevent itself from leaving the stomach through the pylorus. Since the diameter of the pylorus in adults is about 12 mm, the size of the formulation after expansion is no less than, preferably greater than, 12 mm. The brivaracetam sustained-release tablet may have any shape, such as a circle, an oval, an irregular shape, a polygon, etc.

The brivaracetam sustained-release tablet described in the present disclosure has a minimum size of no less than 12 mm after expansion, and even expands to 13 mm or more, thereby preventing it from being discharged from the pylorus of the stomach and ensuring continuous release in the stomach to achieve a therapeutic effect. The brivaracetam sustained-release pharmaceutical composition or the brivaracetam sustained-release tablet described in the present disclosure may expand to any diameter of 12 mm-35 mm, preferably 13 mm-32 mm, and further preferably 13 mm-29 mm.

For example, the size of the brivaracetam sustained-release pharmaceutical composition or the brivaracetam sustained-release tablet after expansion is 28.4 × 15×11.6 mm³, 31.6 × 16.4 × 12.1 mm³, 28 × 14 × 12 mm³, 28.0 × 14.1 × 12.2 mm³, 30.5 × 15.5 × 14.4 mm³, 29.2 × 15.2 × 14.5 mm³, 29.5 × 15.1 × 13.7 mm³, or 29.4 × 15.0 × 13.7 mm³.

### Definitions and Description

In the present disclosure, the term "pharmaceutically acceptable" refers to salts, complexes, solvates, and hydrates of a drug (e.g., brivaracetam), which are, within the scope of sound medical judgment, suitable for use in contact with the tissues of patients without undue toxicity, irritation, allergic response, and the like, commensurate with a reasonable benefit to risk ratio, and effective for their intended use.

The term "solvate" refers to a molecular coordination complex comprising a drug (e.g., brivaracetam) and a stoichiometric or non-stoichiometric amount of one or more pharmaceutically acceptable solvent molecules (e.g., ethanol). When the solvent is closely associated with the drug, the coordination complex formed has a well-defined stoichiometry independent of humidity. However, when the solvent is weakly associated (as in channel solvates and hygroscopic compounds), the solvent content is dependent on humidity and drying conditions; in this case, the coordination complex is generally non-stoichiometric.

The term "hydrate" refers to a solvate comprising a drug (e.g., brivaracetam) and a stoichiometric or non-stoichiometric amount of water.

In the present disclosure, the polyvinyl acetate (PVAc) is a homopolymer of vinyl acetate and generally has a molecular weight M_{w} of about 1 × 10⁵ to about 1 × 10⁶.

The KSR may be available from BASF under the trade name KOLLIDON^{®}SR, known as an 80/20 (w/w) mixture of PVAc and PVP.

In the present disclosure, the polyethylene oxide (PEO) is also known as polyoxirane or polyoxyethylene. Polyethylene oxide is a homopolymer of oxirane and generally has a molecular weight M_{w} of about 1 × 10⁵ to about 1 × 10⁷ or about 1 × 10⁶ to about 1 × 10⁷. Polyethylene oxide is available in various grades based on molecular weight, and may be available from UnionCarbide under the trade name POLYOX^{®}.

The term "more" in "one or more" refers to two or more, e.g., two, three, or four.

The term "more" in "one, two, or more" refers to three or more, e.g., three, four, or five.

The term "therapeutically effective amount" refers to an amount of the active pharmaceutical ingredient of the present disclosure sufficient to effect the intended use (including but not limited to the treatment of a disease as defined below). The therapeutically effective amount may vary depending on the following factors: the intended use (*in vitro* or *in vivo*), or the subject and diseases or conditions being treated, such as the body weight and age of the subject, severity of the diseases or conditions, and route of administration, which may be readily determined by one of ordinary skill in the art. The specific dose will vary depending on the following factors: the selected particular active ingredient, the administration regimen to be followed, whether to administer in combination with other compounds, the schedule of administration, the tissue to be administered, and the physical delivery system carried.

The term "patient" refers to any animal including mammals, preferably mice, rats, other rodents, rabbits, dogs, cats, pigs, cattle, sheep, horses, or primates, and most preferably humans.

The term "matrix-forming agent" includes skeleton materials, disintegrants, and adhesives.

The preferred conditions described above may be combined arbitrarily to obtain preferred embodiments of the present disclosure without departing from the general knowledge in the art.

### Beneficial Effects of Present Disclosure

The brivaracetam sustained-release pharmaceutical composition of the present disclosure has a good sustained-release effect, a stable release rate, and mild side effects. Once-daily administration can improve patient compliance, and an effective therapeutic effect can be achieved by reducing the maximum content of the drug in the blood, prolonging the duration of the drug existing in the body, controlling the effective dose of the drug in the body, and reducing the toxic and side effects of the drug.

Compared with common immediate-release formulations, the brivaracetam sustained-release pharmaceutical composition has the advantages that: (1) the frequency of administration is reduced and a good therapeutic effect can be achieved via sustained release, thereby resulting in improved patient compliance; (2) the retention time of the drug in the stomach can be prolonged, so that the drug is continuously released in the stomach and absorbed at the upper end of the small intestine, thereby achieving a 24-hour continuous effect, stable plasma concentration, and mild adverse effects; (3) patients are prevented from interrupting medication at will, thereby preventing the disease recurrence and the progression of severe complications; and (4) the administration is performed once daily, and the proper specification is selected according to the clinical manifestations, featuring reduced administration frequency, convenience of use, and mild toxic and side effects.

### BRIEF DESCRIPTION OF THE DRAWINGS

FIG. 1 shows the dissolution profiles of the brivaracetam sustained-release pharmaceutical compositions prepared in Examples 1-7 in an acetate buffer at pH 4.5;
FIG. 2 shows the dissolution profiles of the brivaracetam sustained-release pharmaceutical compositions prepared in Examples 7-10 in an acetate buffer at pH 4.5;
FIG. 3 shows the dissolution profiles of the brivaracetam sustained-release pharmaceutical compositions prepared in Examples 7-10 according to a reciprocating cylinder dissolution method;
FIG. 4 shows the mean concentration-time curves of brivaracetam after administration of a test sample (the brivaracetam sustained-release tablet prepared in Example 6)/a control group of original study in Beagle dogs, wherein 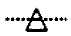 represents the curve of the control group of original study, and 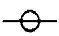 represents the curve of the test sample group;
FIG. 5 shows the mean drug concentration-time curves of brivaracetam in a control group of original study and a test sample group (the brivaracetam sustained-release tablet prepared in Example 7) in healthy subjects, wherein 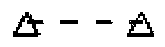 represents the curve of the control group of original study, and 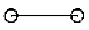 represents the curve of the test sample group.

### DETAILED DESCRIPTION

The technical solutions of the present disclosure are further described in detail below with reference to specific examples. It should be understood that the following examples are merely exemplary illustrations and explanations of the present disclosure and should not be construed as limiting the claimed scope of the present disclosure. All techniques implemented on the basis of the content described above of the present disclosure fall within the claimed scope of the present disclosure.

Unless otherwise stated, the starting materials and reagents used in the following examples are all commercially available products or can be manufactured by using known methods.

Raw and auxiliary materials in the examples comprise the following:
brivaracetam: purchased from Ruyuan HEC Pharmaceutical Co., LTD., with a purity of 98%-102%;
crospolyvinylpyrrolidone: available from Chongqing Star-Tech & JRS Specialty Products Co., Ltd., under the trade name Oridon^{®}XL;
polyethylene oxide: available from DUPONT, under the trade name POLYOX^{™};
KSR: available from BASF, under the trade name KOLLIDON^{®}SR, known as an 80/20 (w/w) mixture of PVAc and PVP.

### Examples 1-4

The formulas of Examples 1-4 are shown in Table 1.

**Table 1**

| Name | Example 1 | | Example 2 | | Example 3 | | Example 4 | | Effect |
|---|---|---|---|---|---|---|---|---|---|
| | Amount (mg/tablet) | Percentage (%) | Amount (mg/tablet) | Percentage (%) | Amount (mg/tablet) | Percentage (%) | Amount (mg/tablet) | Percentage (%) | |
| Brivaracetam | 100 | 9.09 | 100 | 9.09 | 100 | 10.00 | 100 | 10.00 | Active ingredient |
| Polyvinyl acetate povidone mixture | 330 | 30.00 | 330 | 30.00 | 300 | 30.00 | 320 | 32.00 | Skeleton material |
| Sodium carboxymethyl starch | / | / | / | / | / | / | 50 | 5.00 | Disintegrant |
| Crospovidone | 165 | 15.00 | 165 | 15.00 | 170 | 17.00 | 120 | 12.00 | Sweller |
| Carbomer | 44 | 4.00 | 88 | 8.00 | / | / | / | / | Sweller |
| Polyoxyethylene N60K | / | / | / | / | / | / | 150 | 15.00 | Sweller |
| Polyoxyethylene 1105 | / | / | / | / | 160 | 16.00 | / | / | Sweller |
| Hydroxypropyl methylcellulose | 88 | 8.00 | 88 | 8.00 | 70 | 7.00 | 70 | 7.00 | Adhesive |
| Lactose monohydrate | 362 | 32.91 | 230 | 20.91 | / | / | / | / | Filler |
| Pregelatinized starch | / | / | / | / | / | / | 80 | 8.00 | Adhesive |
| Microcrystalline cellulose | / | / | 88 | 8.00 | 190 | 19.00 | 100 | 10.00 | Filler |
| Magnesium stearate | 11 | 1.00 | 11 | 1.00 | 10 | 1.00 | 10 | 1.00 | Lubricant |
| Tablet weight | 1100 | 100.00 | 1100 | 100.00 | 1000 | 100.00 | 1000 | 100.00 | / |
| Film coating powder | 44 | 4.0 | 44 | 4.0 | 40 | 4.0 | 40 | 4.0 | Coating agent |

### Examples 5-7

The formulas of Examples 5-7 are shown in Table 2.

**Table 2**

| Name | Example 5 | | Example 6 | | Example 7 | | Effect |
|---|---|---|---|---|---|---|---|
| | Amount (mg/tablet) | Percentage (%) | Amount (mg/tablet) | Percentage (%) | Amount (mg/tablet) | Percentage (%) | |
| Brivaracetam | 100 | 10.00 | 100 | 10.00 | 100 | 9.09 | Active ingredient |
| Polyvinyl acetate povidone mixture | 320 | 32.00 | 300 | 30.00 | 333 | 30.27 | Skeleton material |
| Sodium carboxymethyl starch | 50 | 5.00 | 60 | 6.00 | 67 | 6.10 | Disintegrant |
| Crospovidone | 120 | 12.00 | 150 | 15.00 | 167 | 15.18 | Sweller |
| Polyoxyethylene N60K | 75 | 7.50 | 150 | 15.00 | 167 | 15.18 | Sweller |
| Polyoxyethylene 1105 | 75 | 7.50 | / | / | / | / | Sweller |
| Hydroxypropyl methylcellulose | 80 | 8.00 | 50 | 5.00 | 56 | 5.09 | Adhesive |
| Pregelatinized starch | 80 | 8.00 | 100 | 10.00 | 110 | 10.00 | Adhesive |
| Microcrystalline cellulose | 90 | 9.00 | 80 | 8.00 | 89 | 8.09 | Filler |
| Magnesium stearate | 10 | 1.00 | 10 | 1.00 | 11 | 1.00 | Lubricant |
| Tablet weight | 1000 | 100.00 | 1000 | 100.00 | 1100 | 100.00 | / |
| Film coating powder | 30 | 3.0 | 40 | 4.0 | 44 | 4.0 | Coating agent |

Preparation process of brivaracetam sustained-release tablets:
Brivaracetam in each of the formulas described above was sieved through a 40-mesh sieve for later use.

Brivaracetam (sieved through a 40-mesh sieve) in each of the formulas described above, together with other auxiliary materials except magnesium stearate, were placed into a hopper mixer and mixed at a mixing speed of 18 rpm for 20 min; the mixed material was sieved using a sizing machine through a sieve having a pore size of 0.8 mm at a rotation speed of 7-12 Hz; the sieved material was then placed into the hopper mixer and mixed at a mixing speed of 18 rpm for 20 min; and finally, magnesium stearate was added, and the mixture was mixed at a mixing speed of 18 rpm for 5 min to give a final mixed powder of brivaracetam.

The final mixed powder of brivaracetam described above was tableted using a 22.0 mm × 10.9 mm melon seed-type die, and the parameters such as tableting speed, pre-tableting pressure, main pressure, and the like were adjusted to ensure that the hardness reached 140 N-240 N for tableting to give a brivaracetam sustained-release non-coated tablet, which was then coated with an aqueous solution of the film coating powder to give a brivaracetam sustained-release tablet.

### Test Example 1

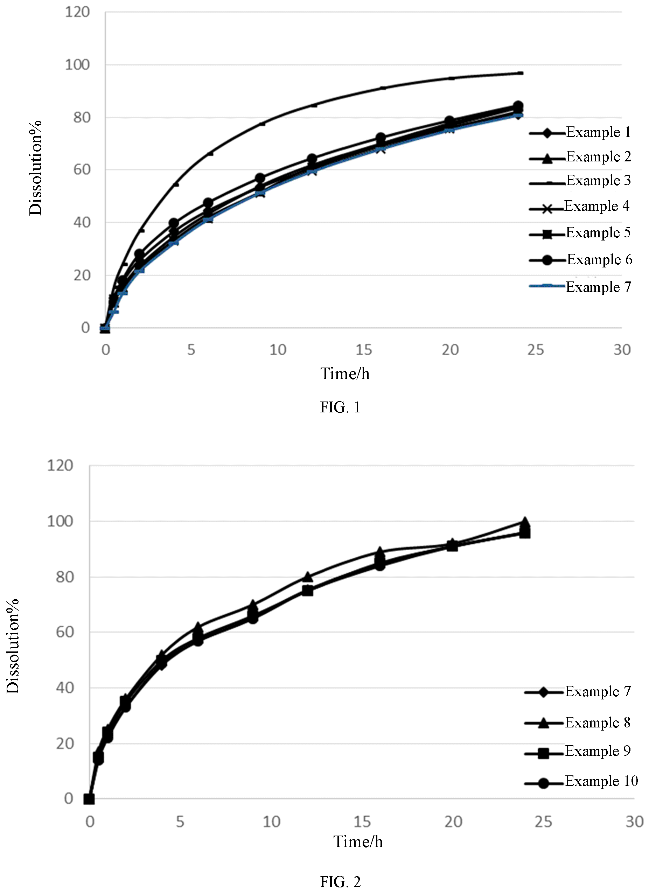

The dissolution profiles and the expansion size after 24 h were detected for the brivaracetam sustained-release tablets obtained in Examples 1-7. The results of the comparative study are as follows:
Dissolution detection method: 900 mL of an acetate buffer at pH 4.5 was used, reference was made to Method 2 in 0931 of Chinese Pharmacopoeia (2020 Edition), and the conditions were 50 rpm and 37±0.5 °C. The dissolution results are shown in Table 3 and FIG. 1.

**Table 3**

| Time/h | Example 1 | Example 2 | Example 3 | Example 4 | Example 5 | Example 6 | Example 7 |
|---|---|---|---|---|---|---|---|
| | Dissolution % | Dissolution % | Dissolution % | Dissolution % | Dissolution % | Dissolution % | Dissolution % |
| 0 | 0 | 0 | 0 | 0 | 0 | 0 | 0 |
| 0.5 | 11.4 | 9.6 | 15.3 | 11.1 | 10.6 | 10.1 | 6.1 |
| 1 | 17.5 | 15.5 | 24 | 14.7 | 14.2 | 18.1 | 12.9 |
| 2 | 25.8 | 23.6 | 36.8 | 22.8 | 22.6 | 28.1 | 21.5 |
| 4 | 36.7 | 34.7 | 54.3 | 33.3 | 33 | 39.7 | 32.2 |
| 6 | 44.6 | 43.4 | 66.1 | 42.1 | 41.6 | 47.7 | 41.2 |
| 9 | 53.5 | 53.9 | 77.4 | 51.4 | 51.5 | 57 | 51.3 |
| 12 | 60.6 | 61.8 | 84.6 | 59.7 | 61.1 | 64.4 | 59.3 |
| 16 | 69.2 | 70 | 91 | 68.1 | 69.5 | 72.3 | 67.9 |
| 20 | 76.1 | 77.7 | 94.9 | 75.8 | 76.9 | 78.8 | 75.1 |
| 24 | 81.2 | 84.3 | 96.8 | 82.2 | 83.7 | 84.6 | 80.8 |

The expansion size after 24 h was detected for the brivaracetam sustained-release tablets obtained in Examples 1-7. The results of the comparative study are shown in Table 4.

**Table 4**

| Size | Example 1 | Example 2 | Example 3 | |
|---|---|---|---|---|
| Long diameter × short diameter × thickness (mm) | 28.4 × 15 × 11.6 | 31.6 × 16.4 × 12.1 | 28 × 14 × 12 | |
| Tablet appearance | Good elasticity, with slight surface shedding observed on the tablet | Good elasticity, with slight surface shedding observed on the tablet | Good elasticity, with serious surface shedding observed on the tablet | |

| Size | Example 4 | Example 5 | Example 6 | Example 7 |
|---|---|---|---|---|
| Long diameter × short diameter × thickness (mm) | 21 × 9 × 11 | 23 × 9 × 12 | 28.0 × 14.1 × 12.2 | 30.5 × 15.5 × 14.4 |
| Tablet appearance | Relatively poor elasticity, with no significant surface shedding observed on the tablet | Good elasticity, with no significant surface shedding observed on the tablet | Very good elasticity, with no significant surface shedding observed on the tablet | Very good elasticity, with no significant surface shedding observed on the tablet |

The above data indicate that the brivaracetam sustained-release tablets prepared by the present disclosure had good sustained-release effects, and after the gastroretentive tablets expanded for 24 h, the size (i.e., long diameter, short diameter, and thickness) of the tablets could all reach the size of the pyloric sphincter of the human stomach.

### Examples 8-10

The formulas of Examples 8-10 are shown in Table 5.

**Table 5**

| Name | Example 8 | | Example 9 | | Example 10 | | Effect |
|---|---|---|---|---|---|---|---|
| | Amount (mg/tablet) | Percentage (%) | Amount (mg/tablet) | Percentage (%) | Amount (mg/tablet) | Percentage (%) | |
| Brivaracetam | 50 | 4.55 | 150 | 13.64 | 200 | 18.18 | Active ingredient |
| Polyvinyl acetate povidone mixture | 345 | 31.37 | 320 | 29.10 | 320 | 29.10 | Skeleton material |
| Sodium carboxymethyl starch | 57 | 5.18 | 70 | 6.36 | 70 | 6.36 | Disintegrant |
| Crospovidone | 158 | 14.36 | 170 | 15.45 | 170 | 15.45 | Sweller |
| Polyoxyethylene N60K | 180 | 16.36 | 140 | 12.73 | 140 | 12.73 | Sweller |
| Hydroxypropyl methylcellulose | 100 | 9.09 | 38 | 3.45 | 38 | 3.45 | Adhesive |
| Pregelatinized starch | 110 | 10.00 | 90 | 8.18 | 90 | 8.18 | Adhesive |
| Microcrystalline cellulose | 89 | 8.09 | 111 | 10.09 | 61 | 5.55 | Filler |
| Magnesium stearate | 11 | 1.00 | 11 | 1.00 | 11 | 1.00 | Lubricant |
| Tablet weight | 1100 | 100 | 1100 | 100.00 | 1100 | 100 | / |
| Film coating powder | 44 | 4.00 | 44 | 4.00 | 44 | 4.00 | Coating material |

### Test Example 2

(I) The dissolution profiles and the expansion size after 24 h were detected for the brivaracetam sustained-release tablets obtained in Examples 7-10. The results of the comparative study are as follows:
Dissolution detection method: 900 mL of an acetate buffer at pH 4.5 was used, reference was made to Method 2 in 0931 of Chinese Pharmacopoeia (2020 Edition), and the conditions were 50 rpm and 37±0.5 °C. The dissolution results are shown in Table 6 and FIG. 2.

**Table 6**

| Time/h | Example 7 | Example 8 | Example 9 | Example 10 |
|---|---|---|---|---|
| | Dissolution% | Dissolution% | Dissolution% | Dissolution% |
| 0 | 0 | 0 | 0 | 0 |
| 0.5 | 6.1 | 10.0 | 9.0 | 9.0 |
| 1 | 12.9 | 16.0 | 15.0 | 15.0 |
| 2 | 21.5 | 23.0 | 24.0 | 23.0 |
| 4 | 32.2 | 33.0 | 34.0 | 33.0 |
| 6 | 41.2 | 41.0 | 42.0 | 42.0 |
| 9 | 51.3 | 53.0 | 52.0 | 51.0 |
| 12 | 59.3 | 60.0 | 59.0 | 57.0 |
| 16 | 67.9 | 69.0 | 67.0 | 65.0 |
| 20 | 75.1 | 76.0 | 73.0 | 71.0 |
| 24 | 80.8 | 82.0 | 79.0 | 78.0 |

The expansion size after 24 h was detected for the brivaracetam sustained-release tablets obtained in Examples 7-10. The results of the comparative study are shown in Table 7:

**Table 7**

| Size | Example 7 | Example 8 | Example 9 | Example 10 |
|---|---|---|---|---|
| Long diameter × short diameter × thickness (mm) | 30.5 × 15.5 × 14.4 | 29.2 × 15.2 × 14.5 | 29.5 × 15.1 × 13.7 | 29.4 × 15.0 × 13.7 |

The brivaracetam sustained-release tablets with specifications of 50 mg-200 mg prepared in Examples 7-10 all had relatively good sustained-release effects, and after the gastroretentive tablets expanded for 24 h, the size (i.e., long diameter, short diameter, and thickness) of the tablets could all reach the size of the pyloric sphincter of the human stomach, demonstrating that the brivaracetam sustained-release tablets with specifications of 50 mg, 100 mg, 150 mg, and 200 mg obtained in the present disclosure have good gastric retention effects and good sustained-release effects; in addition, the dissolution behaviors of the brivaracetam sustained-release tablets with different specifications in the present disclosure are basically consistent, and the sustained-release speed is stable.

(II) According to Method 7 in General Chapter 0931, Dissolution and Drug Release Test, of Chinese Pharmacopoeia, 2020 Edition and under the dissolution conditions that the dissolution media were a hydrochloric acid solution at pH 1.2 and an acetate buffer at pH 4.5, the immersion speed was 20 DPM, the sieve was a 20-mesh polypropylene sieve, and the dripping time was 15 seconds, the release of the brivaracetam sustained-release tablets of the present disclosure in the stomach was further evaluated by adopting a reciprocating cylinder dissolution method, which could simulate the release rate of the tablets in the stomach under the influence of intragastric food or gastric peristalsis. The reciprocating cylinder dissolution method used is shown in Table 8:

**Table 8**

| Reciprocating cylinder dissolution method | | |
|---|---|---|
| Release media | Hydrochloric acid solution at pH 1.2 and acetate buffer at pH 4.5 | |
| Reciprocating speed | 20DPM | |
| First row | pH4.5 | 0.5 h, 1 h, and 2 h |
| Second row | pH1.2 | 4 h |
| Third row | pH4.5 | 6 h |
| Fourth row | pH1.2 | 8 h |
| Fifth row | pH4.5 | 12 h |
| Sixth row | pH1.2 | 16 h, 20 h, and 24 h |

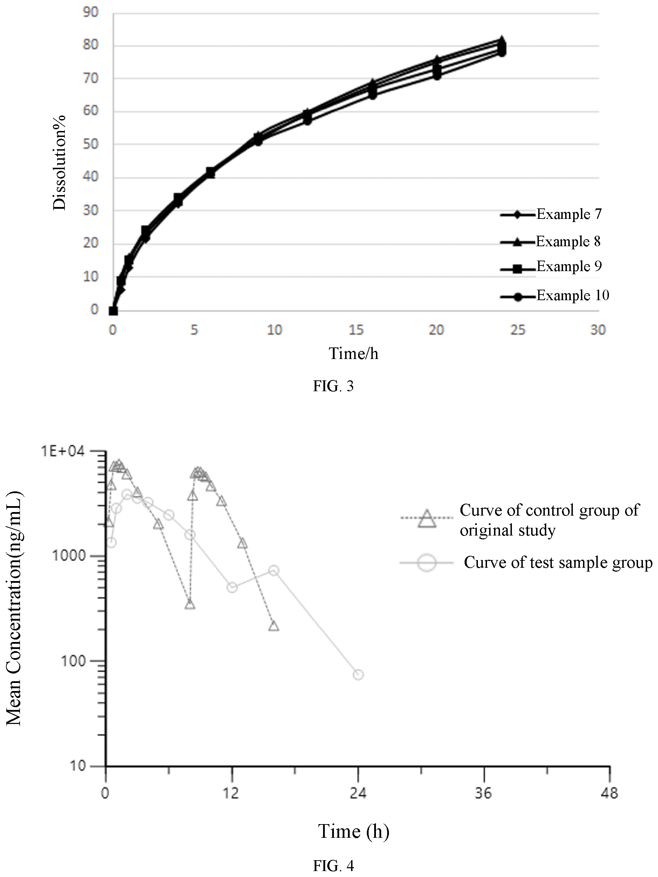

The release rates of the brivaracetam sustained-release tablets with different specifications are shown in Table 9 and FIG. 3:

**Table 9**

| Time/h | Example 7 | Example 8 | Example 9 | Example 10 |
|---|---|---|---|---|
| | Dissolution% | Dissolution% | Dissolution% | Dissolution% |
| 0 | 0 | 0 | 0 | 0 |
| 0.5 | 15.1 | 17.0 | 15.0 | 14.0 |
| 1 | 23.0 | 25.0 | 24.0 | 22.0 |
| 2 | 33.2 | 36.0 | 35.0 | 33.0 |
| 4 | 48.3 | 52.0 | 50.0 | 49.0 |
| 6 | 57.0 | 62.0 | 58.0 | 57.0 |
| 8 | 65.2 | 70.0 | 66.0 | 65.0 |
| 12 | 75.3 | 80.0 | 75.0 | 75.0 |
| 16 | 84.7 | 89.0 | 85.0 | 84.0 |
| 20 | 91.1 | 92.0 | 91.0 | 91.0 |
| 24 | 95.8 | 100.0 | 96.0 | 96.0 |

The results of the study simulating *in vivo* gastric peristalsis and food factors show that the brivaracetam sustained-release tablets of the present disclosure could achieve relatively good sustained-release effects in the stomach, maintain stable plasma concentration, and take effect continuously, thereby achieving the effect of treating diseases.

### Test Example 3. PK Study in Beagle Dogs

In this test, 3 beagle dogs were selected, including 2 males and 1 female. The tablet in Example 6 was selected for the test sample group at a dose of 100 mg/dog/day, and the control group of original study (i.e., the brivaracetam tablet) was administered at a dose of 100 mg/dog/day. The test sample group was administered once, and the control group of original study was administered 1 tablet every 8 h for a total of 2 administrations. Blood samples were collected from the animals in the test sample group at the following time points: before administration (one day before administration) and 0.5 h, 1 h, 2 h, 3 h, 4 h, 6 h, 8 h, 12 h, 16 h, and 24 h after administration; blood samples were collected from the animals in the control group of original study at the following time points: before administration (one day before administration) and 0.25 h, 0.5 h, 0.75 h, 1 h, 1.25 h, 1.5 h, 2 h, 3 h, 5 h, 8 h (performing the second administration after collection), 8.25 h, 8.5 h, 8.75 h, 9 h, 9.25 h, 9.5 h, 10 h, 11 h, 13 h, and 16 h after the first administration. The plasma concentration results are shown in Table 10:

**Table 10**

| Group | Parameter | t_{1/2} | Tₘₐₓ | Cₘₐₓ | AUCₗₐₛₜ | AUC_{inf} | MRTₗₐₛₜ | Vd/F | Cl/F |
|---|---|---|---|---|---|---|---|---|---|
| | | h | h | ng/mL | h·ng/mL | h·ng/mL | h | mL | mL/h |
| Control group of original study | Mean | 1.34 | 2.17 | 8426.92 | 47623.30 | 48054.52 | 6.08 | 4293.29 | 2228.14 |
| | SD | 0.15 | 3.13 | 2568.31 | 14011.79 | 14092.91 | 0.31 | 1274.09 | 607.03 |
| Test sample group | Mean | 1.97 | 2.50 | 4223.31 | 27787.52 | 28171.48 | 4.96 | 10510.46 | 4078.97 |
| | SD | 1.50 | 1.05 | 828.50 | 10991.59 | 10917.65 | 1.81 | 6837.10 | 1718.44 |

The results show that the test sample group (the brivaracetam sustained-release tablet of Example 6) had a prolonged half-life, a lower maximum plasma concentration than that of the control group of original study (the brivaracetam tablet), and a good sustained-release effect. The plasma concentration curves of the control group of original study and the test sample group are shown in FIG. 4.

### Test Example 4. In Vivo PK Study in Healthy Subjects

A PK study on the brivaracetam sustained-release tablet of Example 7 of the present disclosure was carried out in healthy subjects. The original product (50 mg of the brivaracetam tablet) and the test sample (100 mg of the brivaracetam sustained-release tablet) were selected as study drugs. The tablet of Example 7 was selected for the test sample group at a dose of 100 mg/day, and the control group of original study (i.e., the brivaracetam tablet) was administered at a dose of 100 mg/day. The test sample group was administered once, and the control group of original study was administered 1 tablet every 12 h for a total of 2 administrations. Blood samples were collected at scheduled intervals from the control group of original study and the test sample group to determine the plasma concentration. The plasma concentration results are shown in Table 11:

**Table 11**

| Pharmacokinetic parameters | Test sample group Geometric mean | Control group of original study Geometric mean | Geometric mean ratio (test sample group/control group of original study) (%) | Intraindividual coefficient of variation (%) | Geometric mean ratio 90% Confidence interval (%) | |
|---|---|---|---|---|---|---|
| Cmax | 1238.95 | 1280.65 | 96.74 | 12.49 | 89.76 | 104.27 |
| AUClast | 31942.51 | 35029.23 | 91.18 | 4.01 | 89.01 | 93.42 |
| AUCINF_obs | 32378.45 | 35452.01 | 91.33 | 3.74 | 89.29 | 93.41 |

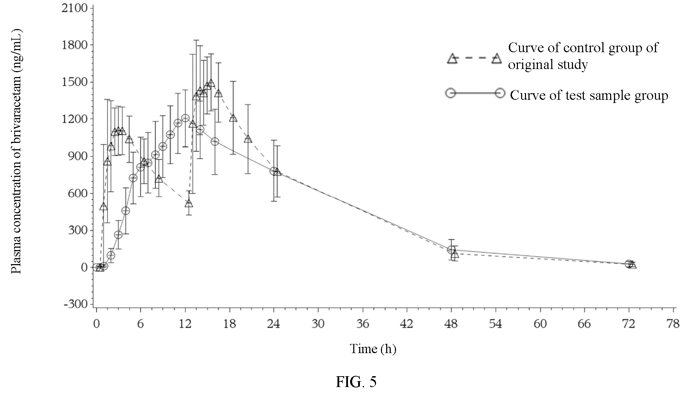

The results show that: the geometric mean ratios of Cₘₐₓ and AUC in the test sample group (the brivaracetam sustained-release tablet) were all within the range of 80%-125%, and the therapeutic effects of 1 brivaracetam sustained-release tablet and 2 brivaracetam tablets were comparable. The brivaracetam sustained-release tablet had a slower drug release rate and more stable plasma concentration, so that the occurrence of adverse effects was reduced while the efficacy was achieved, thereby providing better efficacy, compliance, and safety for patients. The plasma concentration curves of the control group of original study and the test sample group are shown in FIG. 5.

### Test Example 5. Stability Test of Example 7

The tablet product obtained in Example 7 was tested for stability under accelerated conditions (40±2 °C, 75±5% RH) for up to 6 months to detect the related substances, isomers, content, and dissolution (the dissolution was tested by a reciprocating cylinder method according to Test Example 2). The results are shown in Table 12:

**Table 12**

| Product | Test items | 0 day | 1 month | 3 months | 6 months | Acceptance criteria | |
|---|---|---|---|---|---|---|---|
| | Related substance | BWXT-IM-M | Undetectable | Undetectable | Undetectable | Undetectable | ≤0.20% |
| | | Other individual impurities | Undetectable | Undetectable | Undetectable | Undetectable | ≤0.20% |
| | | Total impurity | Undetectable | Undetectable | Undetectable | Undetectable | ≤0.70% |
| | Isomer | BWXT-IM-A | Undetectable | Undetectable | Undetectable | Undetectable | ≤0.20% |
| Tablet of Example 7 | | BWXT-IM-B | 0.09% | 0.09% | 0.11% | 0.11% | ≤2.50% |
| | | BWXT-IM-C | Undetectable | Undetectable | Undetectable | Undetectable | ≤0.20% |
| | Content | | 100.4% | 99.3% | 102.8% | 100.7% | 90.0%-110.0% |
| | Dissolution | 1 h | 23.0% | 25.0% | 23.0% | 24.0% | 30% or less |
| | | 4 h | 48.3% | 50.0% | 48.0% | 50.0% | 35%-60% |
| | | 12 h | 75.3% | 73.0% | 73.0% | 78.0% | 65%-85% |
| | | 24 h | 95.8% | 94.0% | 94.0% | 99.0% | 80% or more |

The above stability test results show that the related substances, isomers, content, and dissolution of the brivaracetam sustained-release tablet of the present disclosure did not exhibit significant changes during the stability test. Thus, the stability of the product is excellent.

### Test Example 6. Comparison of Example 7 with Tablets A and E in the Prior Art CN113908153A

The tablet of Example 7 and tablets A and E in CN113908153A were placed at a high temperature of 60 °C, and samples were collected on days 10 and 30 and tested for the content of related substances. The results are shown in Table 13.

**Table 13. Comparison data of related substances**

| Product | Test items | | 0 day | High temperature, 10 days | High temperature, 30 days | Acceptance criteria |
|---|---|---|---|---|---|---|
| Tablet of Example 7 | Related substance | BWXT-IM-M | Undetectable | Undetectable | Undetectable | ≤0.20% |
| | | Other individual impurities | Undetectable | Undetectable | Undetectable | ≤0.20% |
| | | Total impurity | Undetectable | Undetectable | Undetectable | ≤0.70% |
| Tablet A in CN113908153A | Related substance | BWXT-IM-M | Undetectable | 0.18 | 0.90 | ≤0.20% |
| | | Other individual impurities Total impurity | Undetectable | Undetectable | 0.05 | ≤0.20% |
| | | | Undetectable | 0.18 | 0.95 | ≤0.70% |
| Tablet E in CN113908153A | Related substance | BWXT-IM-M | Undetectable | 0.11 | 0.54 | ≤0.20% |
| | | Other individual impurities | Undetectable | Undetectable | 0.03 | ≤0.20% |
| | | Total impurity | Undetectable | 0.11 | 0.95 | ≤0.70% |

The dissolution profiles and the expansion size after 24 h were tested for the tablet of Example 7 and tablets A and E. The results of the comparative study are shown in Tables 14 and 15.

Dissolution detection method: 900 mL of an acetate buffer at pH 4.5 was used, reference was made to Method 2 in 0931 of Chinese Pharmacopoeia (2020 Edition), and the conditions were 50 rpm and 37±0.5 °C.

**Table 14. Comparison data of expansion sizes of tablets**

| Size | Example 7 | Tablet A in CN113908153A | Tablet E in CN113908153A |
|---|---|---|---|
| Long diameter × short diameter × thickness (mm) | 30.5 × 15.5 × 14.4 | 34.2 × 17.1 × 14.3 | 27.5 × 13.8 × 13.1 |

**Table 15. Comparison data of dissolution speeds**

| Time/h | Example 7 | Tablet A in CN113908153A | Tablet E in CN113908153A |
|---|---|---|---|
| | Dissolution% | Dissolution% | Dissolution% |
| 0 | 0 | 0 | 0 |
| 0.5 | 6.1 | 7.5 | 8.3 |
| 1 | 12.9 | 11.9 | 15.6 |
| 2 | 21.5 | 18.4 | 25.4 |
| 4 | 32.2 | 28.0 | 37.9 |
| 6 | 41.2 | 35.9 | 47.0 |
| 9 | 51.3 | 48.1 | 55.9 |
| 12 | 59.3 | 58. | 63.1 |
| 16 | 67.9 | 68.3 | 70.4 |
| 20 | 75.1 | 76.4 | 77.1 |
| 24 | 80.8 | 83.0 | 85.2 |

Through the above comparison, the tablet obtained in Example 7 of the present disclosure had low impurity content and relatively good stability, while the tablets A and E in CN113908153A had high impurity content and relatively poor stability; the tablet expansion size of Example 7 of the present disclosure met the requirement, while the tablet E in CN113908153A had a relatively small size, which is not conducive to the retention of the tablet in the stomach; the tablet of Example 7 of the present disclosure had a slow dissolution speed and better sustained-release effect. In summary, the tablet of Example 7 of the present disclosure has comprehensive advantages in product stability, tablet expansion size, and sustained-release effect.

The embodiments of the present disclosure have been described above. However, the embodiments described above are not intended to limit the present disclosure. Any modification, equivalent replacement, improvement, and the like made without departing from the spirit and principle of the present disclosure shall fall within the claimed scope of the present disclosure.

## Claims

1. A brivaracetam sustained-release pharmaceutical composition, wherein the brivaracetam sustained-release pharmaceutical composition is a 24-hour sustained-release drug, and the dissolution of the pharmaceutical composition simultaneously meets the following four features:
A) no more than 40% of an active pharmaceutical ingredient is dissolved within 1 hour;
B) 20%-70% of the active pharmaceutical ingredient is dissolved within 4 hours;
C) 60%-90% of the active pharmaceutical ingredient is dissolved within 12 hours; and
D) no less than 70% of the active pharmaceutical ingredient is dissolved within 24 hours;
the active pharmaceutical ingredient is selected from one or more of brivaracetam, a pharmaceutically acceptable complex of brivaracetam, a pharmaceutically acceptable salt of brivaracetam, a pharmaceutically acceptable solvate of brivaracetam, and a pharmaceutically acceptable hydrate of brivaracetam;
preferably, the brivaracetam sustained-release pharmaceutical composition is a 24-hour sustained-release drug, and the dissolution of the pharmaceutical composition simultaneously meets the following four features:
A) no more than 35% of the active pharmaceutical ingredient is dissolved within 1 hour;
B) 30%-70% of the active pharmaceutical ingredient is dissolved within 4 hours;
C) 60%-85% of the active pharmaceutical ingredient is dissolved within 12 hours; and
D) no less than 75% of the active pharmaceutical ingredient is dissolved within 24 hours;
preferably, the brivaracetam sustained-release pharmaceutical composition is a 24-hour sustained-release drug, and the dissolution of the pharmaceutical composition simultaneously meets the following four features:
A) no more than 30% of the active pharmaceutical ingredient is dissolved within 1 hour; preferably, no more than 30% of brivaracetam or the pharmaceutically acceptable salt thereof is dissolved within 1 hour;
B) 35%-60% of the active pharmaceutical ingredient is dissolved within 4 hours; preferably, 35%-60% of brivaracetam or the pharmaceutically acceptable salt thereof is dissolved within 4 hours;
C) 65%-85% of the active pharmaceutical ingredient is dissolved within 12 hours; preferably, 65%-85% of brivaracetam or the pharmaceutically acceptable salt thereof is dissolved within 12 hours; and
D) no less than 80% of the active pharmaceutical ingredient is dissolved within 24 hours; preferably, no less than 80% of brivaracetam or the pharmaceutically acceptable salt thereof is dissolved within 24 hours.

2. A brivaracetam sustained-release pharmaceutical composition, wherein the brivaracetam sustained-release pharmaceutical composition comprises an active pharmaceutical ingredient and a pharmaceutical excipient, wherein the pharmaceutical excipient is selected from one or more of a skeleton material, a disintegrant, a sweller, an adhesive, a filler, and a lubricant; the active pharmaceutical ingredient is selected from one or more of the following: brivaracetam, a pharmaceutically acceptable complex of brivaracetam, a pharmaceutically acceptable salt of brivaracetam, a pharmaceutically acceptable solvate of brivaracetam, and a pharmaceutically acceptable hydrate of brivaracetam;
preferably, the brivaracetam sustained-release pharmaceutical composition has the dissolution features as claimed in claim 1.

3. The brivaracetam sustained-release pharmaceutical composition according to claim 2, wherein
the active pharmaceutical ingredient is brivaracetam;
and/or,
the active pharmaceutical ingredient has a weight percentage of 2.00%-50.00%, preferably 3.00%-20.00%, for example, 4.00%, 4.55%, 5.00%, 6.00%, 7.00%, 8.00%, 9.00%, 9.09%, 10.00%, 11.00%, 12.00%, 13.00%, 13.64%, 14.00%, 15.00%, 16.00%, 17.00%, 18.00%, 18.18%, 19.00%, 20.00%, 25.00%, 30.00%, 35.00%, 40.00%, or 50.00%; the weight percentage refers to a percentage of the weight of the active pharmaceutical ingredient in the total weight of the brivaracetam sustained-release pharmaceutical composition;
and/or,
the brivaracetam sustained-release pharmaceutical composition comprises microcrystalline cellulose and/or hydroxypropyl methylcellulose;
preferably, the microcrystalline cellulose has a weight percentage of 1.00%-60.00%, for example, 2.00%-40.00% or 5.00%-15.00%; the weight percentage refers to a percentage of the weight of the microcrystalline cellulose in the total weight of the brivaracetam sustained-release pharmaceutical composition;
preferably, the hydroxypropyl methylcellulose has a weight percentage of 1.00%-40.00%, for example, 2.00%-20.00%; the weight percentage refers to a percentage of the weight of the hydroxypropyl methylcellulose in the total weight of the brivaracetam sustained-release pharmaceutical composition;
and/or,
the brivaracetam sustained-release pharmaceutical composition comprises 25 mg to 200 mg, preferably 25 mg, 50 mg, 100 mg, 150 mg, or 200 mg, of brivaracetam.

4. The brivaracetam sustained-release pharmaceutical composition according to claim 2, wherein
the skeleton material is selected from one or more of polyvinyl acetate povidone mixture, sodium alginate, ethylcellulose, cellulose acetate, cellulose acetate butyrate, poloxamer, polacrilin potassium, acrylic resin, and polyvinyl alcohol, preferably polyvinyl acetate povidone mixture;
and/or,
the disintegrant is selected from one or more of sodium carboxymethyl starch, low-substituted hydroxypropylcellulose, corn starch, and croscarmellose sodium, preferably sodium carboxymethyl starch; and/or,
the sweller is selected from one or more of polyoxyethylene, crospovidone, carbomer, sodium carboxymethylcellulose, and calcium carboxymethylcellulose, preferably one or more of polyoxyethylene, crospovidone, and carbomer, and more preferably polyoxyethylene and/or crospovidone;
and/or,
the adhesive is selected from one or more of hydroxypropyl methylcellulose, pregelatinized starch, copovidone, hydroxyethylcellulose, and dextrin, preferably hydroxypropyl methylcellulose and/or pregelatinized starch; and/or,
the filler is selected from one or more of microcrystalline cellulose, silicified microcrystalline cellulose, lactose, mannitol, and sorbitol, preferably microcrystalline cellulose and/or lactose;
and/or,
the lubricant is selected from one or more of magnesium stearate, calcium stearate, and sodium stearyl fumarate, preferably magnesium stearate.

5. The brivaracetam sustained-release pharmaceutical composition according to claim 2 or 4, wherein
the skeleton material has a weight percentage of 5.00%-60.00%, preferably 20.00%-40.00%, for example, 20.00%, 25.00%, 29.10%, 30.00%, 30.27%, 31.37%, 32.00%, 33.00%, 34.00%, 35.00%, or 40.00%; the weight percentage refers to a percentage of the weight of the skeleton material in the total weight of the brivaracetam sustained-release pharmaceutical composition;
and/or,
the disintegrant has a weight percentage of 1.00%-10.00%, preferably 2.00%-8.00%, for example, 3.00%, 4.00%, 5.00%, 5.18%, 6.00%, 6.10%, 6.36%, or 7.00%; the weight percentage refers to a percentage of the weight of the disintegrant in the total weight of the brivaracetam sustained-release pharmaceutical composition; and/or,
the sweller has a weight percentage of 5.00%-60.00%, preferably 10.00%-40.00%, for example, 10.00%, 15.00%, 17.00%, 19.00%, 20.00%, 23.00%, 25.00%, 27.00%, 28.18%, 30.00%, 30.36%, 30.72%, 33.00%, 35.00%, or 40.00%; the weight percentage refers to a percentage of the weight of the sweller in the total weight of the brivaracetam sustained-release pharmaceutical composition;
and/or,
the adhesive has a weight percentage of 1.00%-40.00%, preferably 5.00%-20.00%, for example, 2.00%, 3.00%, 4.00%, 5.00%, 7.00%, 8.00%, 10.00%, 11.63%, 15.00%, 15.09%, 16.00%, 19.09%, 20.00%, 21.00%, 22.00%, 23.00%, 24.00%, 25.00%, or 30.00%; the weight percentage refers to a percentage of the weight of the adhesive in the total weight of the brivaracetam sustained-release pharmaceutical composition;
and/or,
the filler has a weight percentage of 1.00%-60.00%, preferably 5.00%-40.00%, for example, 2.00%, 3.00%, 4.00%, 5.00%, 5.55%, 6.00%, 7.00%, 8.00%, 8.09%, 9.00%, 10.00%, 10.09%, 15.00%, 19.00%, 20.00%, 25.00%, 28.91%, 30.00%, 32.91%, 35.00%, or 40.00%; the weight percentage refers to a percentage of the weight of the filler in the total weight of the brivaracetam sustained-release pharmaceutical composition;
and/or,
the lubricant has a weight percentage of 1.00%-5.00%, preferably 1.00%-2.00%, for example, 1.00%; the weight percentage refers to a percentage of the weight of the lubricant in the total weight of the brivaracetam sustained-release pharmaceutical composition.

6. The brivaracetam sustained-release pharmaceutical composition according to any one of claims 2-5, wherein the brivaracetam sustained-release pharmaceutical composition consists of an active pharmaceutical ingredient, a skeleton material, a disintegrant, a sweller, an adhesive, a filler, and a lubricant;
preferably, the active pharmaceutical ingredient is brivaracetam or a pharmaceutically acceptable salt thereof; and/or, the skeleton material is polyvinyl acetate povidone mixture;
and/or, the disintegrant is sodium carboxymethyl starch;
and/or, the sweller is selected from one or more of polyoxyethylene, crospovidone, and carbomer;
and/or, the adhesive is selected from hydroxypropyl methylcellulose and/or pregelatinized starch;
and/or, the filler is selected from microcrystalline cellulose and/or lactose;
and/or, the lubricant is selected from magnesium stearate;
preferably, the brivaracetam sustained-release pharmaceutical composition consists of the following components in percentage by weight: 2.00%-50.00% of brivaracetam, 5.00%-60.00% of the skeleton material,1.00%-10.00% of the disintegrant, 5.00%-60.00% of the sweller, 1.00%-40.00% of the adhesive, 1.00%-60.00% of the filler, and 1.00%-5.00% of the lubricant, wherein the percentage by weight refers to a percentage of the weight of an individual component in the total weight of the brivaracetam sustained-release pharmaceutical composition;
or the brivaracetam sustained-release pharmaceutical composition consists of the following components in percentage by weight: 3.00%-20.00% of brivaracetam, 20.00%-40.00% of the skeleton material, 2.00%-8.00% of the disintegrant, 10.00%-40.00% of the sweller, 5.00%-20.00% of the adhesive, 5.00%-40.00% of the filler, and 1.00%-2.00% of the lubricant, wherein the percentage by weight refers to a percentage of the weight of an individual component in the total weight of the brivaracetam sustained-release pharmaceutical composition;
preferably, the brivaracetam sustained-release pharmaceutical composition consists of the following formulations:
formulation 1: brivaracetam, KSR, crospovidone, carbomer, lactose, hydroxypropyl methylcellulose, and magnesium stearate;
formulation 2: brivaracetam, KSR, crospovidone, carbomer, lactose, hydroxypropyl methylcellulose, microcrystalline cellulose, and magnesium stearate;
formulation 3: brivaracetam, KSR, crospovidone, polyoxyethylene, microcrystalline cellulose, hydroxypropyl methylcellulose, and magnesium stearate;
formulation 4: brivaracetam, KSR, sodium carboxymethyl starch, crospovidone, polyoxyethylene, microcrystalline cellulose, pregelatinized starch, hydroxypropyl methylcellulose, and magnesium stearate;
formulation 5: brivaracetam, KSR, sodium carboxymethyl starch, crospovidone, polyoxyethylene (e.g., polyoxyethylene N60K and/or polyoxyethylene 1105), microcrystalline cellulose, pregelatinized starch, hydroxypropyl methylcellulose, and magnesium stearate;
further preferably, the brivaracetam sustained-release pharmaceutical composition is selected from any one of the following formulas:
formula 1: 9.09% brivaracetam, 30.00% polyvinyl acetate povidone mixture, 15.00% crospovidone, 4.00% carbomer, 8.00% hydroxypropyl methylcellulose, 32.91% lactose monohydrate, and 1.00% magnesium stearate;
formula 2: 9.09% brivaracetam, 30.00% polyvinyl acetate povidone mixture, 15.00% crospovidone, 8.00% carbomer, 8.00% hydroxypropyl methylcellulose, 20.91% lactose monohydrate, 8.00% microcrystalline cellulose, and 1.00% magnesium stearate;
formula 3: 10.00% brivaracetam, 30.00% polyvinyl acetate povidone mixture, 17.00% crospovidone, 16.00% polyoxyethylene 1105, 7.00% hydroxypropyl methylcellulose, 19.00% microcrystalline cellulose, and 1.00% magnesium stearate;
formula 4: 10.00% brivaracetam, 32.00% polyvinyl acetate povidone mixture, 5.00% sodium carboxymethyl starch, 12.00% crospovidone, 15.00% polyoxyethylene N60K, 7.00% hydroxypropyl methylcellulose, 8.00% pregelatinized starch, 10.00% microcrystalline cellulose, and 1.00% magnesium stearate;
formula 5: 10.00% brivaracetam, 32.00% polyvinyl acetate povidone mixture, 5.00% sodium carboxymethyl starch, 12.00% crospovidone, 7.50% polyoxyethylene N60K, 7.50% polyoxyethylene 1105, 8.00% hydroxypropyl methylcellulose, 8.00% pregelatinized starch, 9.00% microcrystalline cellulose, and 1.00% magnesium stearate;
formula 6: 10.00% brivaracetam, 30.00% polyvinyl acetate povidone mixture, 6.00% sodium carboxymethyl starch, 15.00% crospovidone, 15.00% polyoxyethylene N60K, 5.00% hydroxypropyl methylcellulose, 10.00% pregelatinized starch, 8.00% microcrystalline cellulose, and 1.00% magnesium stearate;
formula 7: 9.09% brivaracetam, 30.27% polyvinyl acetate povidone mixture, 6.10% sodium carboxymethyl starch, 15.18% crospovidone, 15.18% polyoxyethylene N60K, 5.09% hydroxypropyl methylcellulose, 10.00% pregelatinized starch, 8.09% microcrystalline cellulose, and 1.00% magnesium stearate;
formula 8: 4.55% brivaracetam, 31.37% polyvinyl acetate povidone mixture, 5.18% sodium carboxymethyl starch, 14.36% crospovidone, 16.36% polyoxyethylene N60K, 9.09% hydroxypropyl methylcellulose, 10.00% pregelatinized starch, 8.09% microcrystalline cellulose, and 1.00% magnesium stearate;
formula 9: 13.64% brivaracetam, 29.10% polyvinyl acetate povidone mixture, 6.36% sodium carboxymethyl starch, 15.45% crospovidone, 12.73% polyoxyethylene N60K, 3.45% hydroxypropyl methylcellulose, 8.18% pregelatinized starch, 10.09% microcrystalline cellulose, and 1.00% magnesium stearate;
formula 10: 18.18% brivaracetam, 29.10% polyvinyl acetate povidone mixture, 6.36% sodium carboxymethyl starch, 15.45% crospovidone, 12.73% polyoxyethylene N60K, 3.45% hydroxypropyl methylcellulose, 8.18% pregelatinized starch, 5.55% microcrystalline cellulose, and 1.00% magnesium stearate; the percentage refers to a percentage of the weight of each component in the total weight of the brivaracetam sustained-release pharmaceutical composition.

7. A brivaracetam sustained-release tablet, wherein the brivaracetam sustained-release tablet consists of a tablet core and a coating agent, wherein the tablet core comprises the sustained-release pharmaceutical composition according to any one of claims 1-6;
the coating agent is a film coating powder;
and/or,
the content of the coating agent is 0%-10.0%, for example, 4.00% or 3.00%, wherein the content refers to a percentage of the weight of the coating agent in the total weight of the tablet core.

8. A preparation method for the brivaracetam sustained-release pharmaceutical composition according to any one of claims 1-6 or the brivaracetam sustained-release tablet according to claim 7, wherein the preparation method is a direct powder compression method, a dry granulation method, a wet granulation method, or a fluidized granulation method.

9. Use of the brivaracetam sustained-release pharmaceutical combination according to any one of claims 1-6 or the brivaracetam sustained-release tablet according to claim 7 for manufacturing a medicament.

10. The use according to claim 9, wherein the medicament is used for treating and/or preventing the following diseases: epilepsy, Parkinson's disease, dyskinesia, migraine, tremor, essential tremor, bipolar disorder, chronic diseases, neuropathic pain, bronchial asthma, or allergic bronchial asthma;
preferably, the medicament is used for treating and/or preventing partial-onset seizures in patients aged 16 years or more.

11. A method for preventing and/or treating a condition or disease responsive to brivaracetam, comprising administering to a patient a therapeutically effective amount of the brivaracetam sustained-release pharmaceutical composition according to any one of claims 1-6 or the brivaracetam sustained-release tablet according to claim 7, wherein
preferably, the condition or disease is selected from epilepsy (e.g., partial-onset seizures), Parkinson's disease, dyskinesia, migraine, tremor, essential tremor, bipolar disorder, chronic diseases, neuropathic pain, bronchial asthma, and allergic bronchial asthma.
